# EUROPEAN PATENT SPECIFICATION

(11) **EP 1 511 419 B1**
(45) Date of publication and mention of the grant of the patent: **20.08.2008**
(21) Application number: 03741635.1
(22) Date of filing: 12.06.2003
(51) Int. Cl.: A61B 5/103, A61B 5/107

(54) **METHOD FOR CHECKING A DIABETIC FOOT**
VERFAHREN ZUR ÜBERPRÜFUNG VON EINEM DIABETISCHEN FUSS
PROCEDE D'EXAMEN D'UN PIED D'UNE PERSONNE DIABETIQUE

(30) Priority: 12.06.2002 NL 1020839
(43) Date of publication of application: 09.03.2005
(73) Proprietor: Medavinci Development B.V., 1076 AZ Amsterdam (NL)
(72) Inventor: BIESBROUCK, Gerardus, Majella, NL-2082 HG Santpoort-Zuid (NL); DEN OUDEN, Arie, Huibrecht, NL-4697 DG St. Annaland (NL)
(74) Representative: van Westenbrugge, Andries
(86) International application number: PCT/NL2003/000424
(87) International publication number: WO 2004/002309

(56) References cited:
- EP-A- 0 558 975
- EP-A- 0 829 209
- EP-A- 0 895 746
- WO-A-91/17708
- WO-A2-01/87143
- DE-A- 4 337 608
- FR-A- 2 637 488
- FR-A- 2 730 403
- FR-A- 2 789 883
- US-A- 4 534 365
- US-A- 4 538 353
- US-A- 4 858 621
- US-A- 4 897 924
- US-B1- 6 205 230
- RAJBHANDARI S.M.; HARRIS N.D.: 'digital imaging: an accurate and easy method of measuring foot ulcers' DIABETIC MEDECINE: A JOURNAL OF THE BRITISH DIABETIC ASSOCIATION vol. 16, no. 4, April 1999, ENGLAND, pages 339 - 342
- LAJI K. ET AL: 'Locally developed digital image archive for diabetic foot clinic: a DGH experience' PRACTICAL DIABETES INT vol. 18, September 2001, pages 231 - 234
- ARMSTRONG D.G.; LAVERY L.A.; LISWOOD P.J.: 'Infrared dermal thermometry for the high-risk diabetic foot' PHYSICAL THERAPY vol. 77, February 1997, USA, pages 169 - 177
- WIRTHLIN D.J. ET AL: 'Telemedicine in vascular surgery: Feasibility of digital imaging for remote management of wounds' JOURNAL OF VASCULAR SURGERY vol. 27, no. 6, June 1998, ST. LOUIS, MO, US, pages 1089 - 1100, XP005128641

## Description

The present invention relates to method for checking a diabetic foot. The invention relates in particular to recording the sole of a foot, comprising image-recording means for making an electronic image of the sole of the foot.

Approximately fifteen per cent of diabetic patients sustain a foot wound at one time in their life. A foot wound is often chronic and difficult to treat. Diabetic patients often have little or no feeling in their feet. This phenomenon is known as 'diabetic foot'. A foot wound in a patient, if not noticed in time, can lead to amputation of a leg. Forty to seventy per cent of all lower leg amputations are performed on diabetic patients. Early treatment of foot wounds in these patients is therefore very important. Recording systems for analyzing a diabetic foot generally use pressure sensors to detect problems. DE 43 37 608 A1 discloses a system in which pressure sensors are used. The measured values of the pressure sensors are sent to a computer for analysis of the data. Such a device is relatively expensive and must be operated by an expert. This means that such a device is suitable only for use in a clinic or in a hospital. Moreover, such a measuring apparatus is not suitable for detecting small wounds.

A method of the type described above is known from WO 91/17708. The used device comprises a computer and a visual display unit for examining measured results. The measured results contain information on the curve of the sole of the foot, and are used for making a suitable shoe sole. The recorded image of the sole of the foot is converted into a contour map. The recorded image of the sole of the foot as such is used no further.

A method for checking the sole of a foot of a diabetic patient is described in the publication "Locally developed digital image archive for diabetic foot clinic: a DGH experience" PRACTICAL DIABETES INT, vol. 18, September 2001 (2001-09), pages 231-234, K.LAJI, J. KUMAR, J. BISHOP, M. PAGE. The used system is run on a portable computer in a foot clinic along with a digital camera to photograph feet lesions. Reference is made to the fact that the buil in data transfer capabilities of the used image database software enable future integration into hospital-wide information systems.

It is an object of the present invention to make it possible to record the sole of a foot in a patient at home by means of a relatively simple device, in the case of which the sole of the foot can be diagnosed remotely by an expert.

To that end, the present invention provides a method having the features of claim 1.

According to a preferred embodiment, the device used in the method contains no sensors, but only image-recording means, for recording the sole of the foot. Sending the electronic image makes it possible for a remote monitor, for example a doctor, to examine the sole of the foot and take action if necessary. Sending images regularly ensures that a deterioration in conditions of the sole of the foot can be noticed in time.

The device used in the method comprises a transparent supporting surface, for supporting a foot. A patient can then easily place his/her feet on the supporting surface, after which a image of the sole of the foot is made.

In one embodiment the electronic image comprises a color photocopy of the sole of the foot. Color information is desirable during the examination of a diabetic foot.

The device may comprise a connection for a television and/or a computer. In this case a patient himself/herself can look at a image using commonly available equipment, such as a TV or PC.

Diabetic patients often have impaired sight. For that reason, the image-recording means in one embodiment of the invention comprise tuning means to permit zooming in or zooming out the sole of the foot. With this device, a monitor can study the sole of the foot in very great detail, with the result that small wounds are more likely to be detected.

The device used in the method may further comprise a regulating device, which is designed to communicate with the image-recording means, in order to control the image-recording means remotely. The regulating device can be, for example, a remote control, so that the patient standing on the device can zoom in or zoom out, or can record and send a image using the remote control.

The image-recording means preferably comprise a digital camera. Such a camera can make images that can be sent in a simple manner by way of a digital channel.

According to the invention the transparent supporting surface is placed on a box-shaped construction containing the image-recording means. In this way the device is very practical and the image-recording means are protected.

The device may contain switching means, which are designed to activate the image-recording means after pressure has been exerted upon the supporting surface. All a patient now has to do is stand on the device and a image of the sole of the foot or soles of the feet will then be taken.

Preferably, the sending means are designed for sending a digital photocopy by way of a digital network, such as the Internet. The sending means comprise, for example, a modem for sending digital signals by way of a telephone line. A image can then be sent, via the Internet for example, directly to a doctor or another suitable person. This can render the visit to the doctor unnecessary. It will be clear that the modem can be a wireless modem or telephone. In this case the device will be a stand-alone apparatus, which can also be used outdoors.

The checking of the sole of the foot comprises the following steps:
d) detecting any small wounds that may be present on the sole of the foot;
e) comparing the sole of the foot with any previous check that may have been made.

The person who is having the sole of the foot checked can preferably also look at the electronic image himself/herself on a visual display unit that can be connected.

Further advantages and features of the present invention will become clear on the basis of a description of a preferred embodiment, with reference to the appended drawings, in which:
Fig. 1 shows a side view of a device to be used by the method according to the invention;
Fig. 2 is a top view of a device to be used by the method according to the invention.

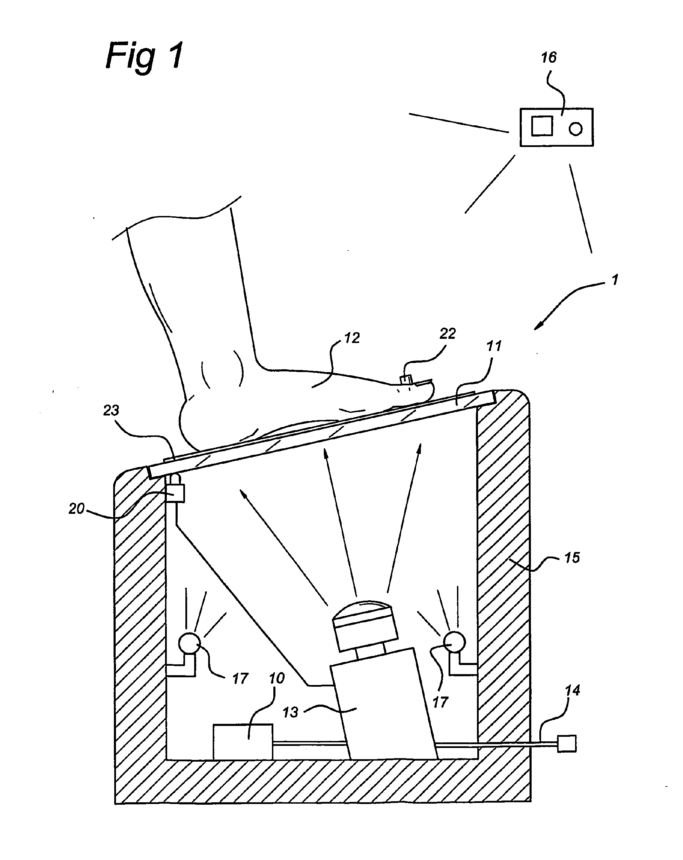

Figure 1 shows a side view of a configuration of the used device. The device 1 consists of a transparent supporting surface 11, which is made of, for example, glass. A foot 12 can be placed on said supporting surface 11. The device 1 comprises a video camera 13, which is directed at the transparent supporting surface 11. The transparent supporting surface 11 is supported on a box-shaped construction 15. The device 1 comprises sending means 10, for example a modem, for sending image information to a remote monitor. The sending means 10 are connected to an output port of the video camera 13. The sending means can comprise a modem, which can be connected to a computer network, such as the Internet, so that the recorded image can be sent to, for example, a doctor. The latter can then examine the image and, if necessary, invite the user of the device 1 for further examination. An address of the checking doctor to which the data is to be sent may be input in advance, possibly by a supplier. Since the data concerned is private and confidential, the data is preferably sent in encoded form. The device can also comprise switching means 20, such as a switch, which is connected to the camera 13, and is designed in such a way that when pressure is exerted upon the supporting surface 11, the camera 13 is activated, possibly after a delay. In one embodiment the device 1 comprises a (wireless) connection 14 of the video camera 13 to a display means such as, for example, a television. The device 1 also has a regulating device 16, such as a remote control, for the remote control of the video camera 13. The device 1 comprises light means 17 for illuminating the sole of the foot.

If a user of the device 1 wishes to see the images himself/herself, he/she must connect the device 1 to, for example, a TV. If the connection 14 is a cable, the user must connect the cable to an input port of the TV. In the case of wireless communication, the wireless connection 14 only has to be activated. The TV and the video camera 13 then have to be switched on and the correct channel selected on the TV; this is usually the video channel. The user then places one foot or both feet on the supporting surface 11. The supporting surface 11 preferably forms an angle with the horizontal plane, so that patients sitting on a chair find it easy to place their feet on the device 1 and do not exert too much pressure on the supporting surface 11. When the feet have been placed, the soles of the feet become visible on the TV screen. The user can now increase or reduce the size of the image, if desired, using the regulating device 16. In another configuration the sole of a foot can be displayed on the visual display unit of a computer. If the correct software and control programs have been loaded, the user can examine the images and store them on a hard disk of the computer, if desired. The video camera 13 can be a digital video camera. An electronic photographic camera or a digital photographic camera can also be used. The image-recording means could also be a flatbed scanner, which can be connected to a PC.

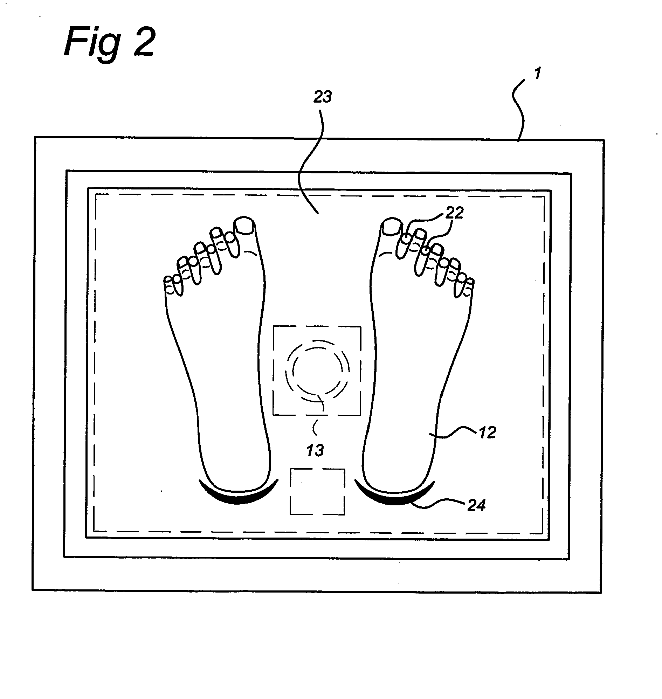

The device 1 preferably comprises a toe-spreading device, which consists of a preferably transparent mat 23 on which elevations 22 are provided. The way in which the toe-spreading device works can be seen better in Figure 2. The toe-spreading device can be placed on the transparent supporting surface 11, as can be seen in Figure 1. The toe-spreading device preferably comprises several elevations 22, which are, for example, the shape of a cylinder. The toes of a user will have to be placed vertically on the device, so that when they come into contact with the elevations 22, they are moved apart. This produces more space between the individual toes. In this example the toe-spreading device consists of 4 elevations per foot.

The device can also comprise positioning means 24 for positioning the foot 12. A user can consequently position the feet 12 in such a way that a good view of them is obtained. The positioning means 24 can consist of only two curved lines placed on the supporting surface 11 or on the mat 23, but they could also be two possibly curved, upright arches, against which the heels have to be placed.

It will be understood on reading of the above that variants will immediately spring to mind in the person skilled in the Such variants are considered to lie within the scope of the invention as defined in the appended claims. The camera can, for example, be set to record several images after activation and to send those that are sharpest. It is also conceivable for light means with light of a certain wavelength to be used. Infrared light can be used, for example, in order to make a temperature profile of the sole of a foot. This temperature profile may, if desired, be sent together with a true copy of the sole of a foot, so that a doctor has additional information at his disposal. It is also possible for monochromatic light to be used. Drying of the sole of the foot can, for example, be detected most easily with the aid of ultraviolet light. Other wavelengths are also possible, so that, for example, underlying structures in the sole of the foot can be made visible. In order to alert the patient to the fact that a photograph has to be made and sent, the device can also comprise alarm means, which alert the patient at regular times about the need for a check. This guarantees regular sending of a photograph to a checking point. In addition, it is conceivable for the device to be equipped for the sending of identification data, so that it is clear to any doctor from whom the photograph has come.

## Claims

1. Method for checking the sole of a foot of a diabetic patient, comprising the steps of:
a) providing a device for recording the sole of a foot, said device comprising image-recording means (13) for making an electronic image of the sole of the foot, sending means (10) for sending the electronic image to a remote monitor, a transparent supporting surface for supporting the foot where the transparent supporting surface is placed on a box-shaped construction containing the image recording means, and positioning means (24) on the supporting surface for positioning the foot relative to said image recording means (13) so that a good view is obtained,
b) making an electronic image of the sole of a foot by the patient himself at the patient's home with the aid of said device;
c) sending the electronic image to the remote monitor via the sending means (10) and a digital network;
d) checking of the sole of the foot by the remote monitor, wherein said checking of the sole of the foot comprises:
d1) detecting any small wounds that may be present on the sole of the foot;
d2) comparing the sole of the foot with any previous check that may have been made.

2. Method according to claim 1, wherein said electronic image comprises a color photocopy of the sole of the foot.

3. Method according to claim 1, wherein said image recording means comprises a digital camera.

4. Method according to claim 1, wherein said sending means comprises a modem.

5. Method according to claim 1, wherein said device comprises alarm means for alerting the patient at regular times about the meed for a check.

## Patentansprüche

1. Verfahren zur Kontrolle der Fußsohle bei einem zuckerkranken Patienten, umfassend die Schritte
a) Bereitstellung einer Vorrichtung zur Erfassung der Fußsohle, wobei die Vorrichtung ein Bildaufzeichnungsmittel (13) zur Herstellung eines elektronischen Bildes der Fußsohle, ein Sendemittel (10) zum Senden des elektronischen Bildes an einen Fernmonitor, eine transparente Stützfläche zum Stützen des Fußes, wobei die transparente Stützfläche auf eines kastenförmige, das Bildaufzeichnungsmittel enthaltende Anlage angeordnet wird, und ein Positioniermittel (24) auf der Stützfläche zum Positionieren des Fußes in Relation zu dem Bildaufzeichnungsmittel (13), so dass ein guter Überblick erhalten wird, umfasst,
b) Herstellung eines elektronischen Bildes der Fußsohle durch den Patienten selbst in seinem Zuhause mit Hilfe dieser Vorrichtung,
c) Senden des elektronischen Bildes über das Sendemittel (10) an den Fernmonitor und ein digitales Netzwerk,
d) Kontrolle der Fußsohle am Fernmonitor, wobei die Kontrolle der Fußsohle umfasst.
d1) Erfassen kleiner Wunden, die auf der Fußsohle vorhanden sein können,
d2) Vergleich der Fußsohle mit jeder früheren Kontrolle, die durchgeführt worden sein kann.

2. Verfahren nach Anspruch 1, worin das elektronische Bild eine Farbkopie der Fußsohle umfasst.

3. Verfahren nach Anspruch 1, worin das Bildaufzeichnungsmittel eine Digitalkamera umfasst.

4. Verfahren nach Anspruch 1, worin das Sendemittel ein Modem umfasst.

5. Verfahren nach Anspruch 1, worin die Vorrichtung ein Alarmmittel umfasst, das den Patienten zu regelmäßigen Zeitpunkten warnend auf die Notwendigkeit einer Kontrolle hinweist.

## Revendications

1. Procédé d'examen du pied d'un patient diabétique, comprenant les étapes comprenant :
(a) la fourniture d'un dispositif d'enregistrement de la plante d'un pied, ledit dispositif comprenant un moyen d'enregistrement d'image (13) pour produire une image électronique de la plante du pied, un moyen d'envoi (10) pour envoyer l'image électronique à un moniteur distant, une surface de support transparente pour supporter le pied, ladite surface de support transparente étant placée sur une construction de type boîte contenant le moyen d'enregistrement d'image, et un moyen de positionnement (24) sur la surface de support pour positionner le pied par rapport audit moyen d'enregistrement d'image (13) de sorte qu'une vue satisfaisante soit obtenue,
(b) la réalisation d'une image électronique de la plante d'un pied par le patient lui-même au domicile du patient à l'aide dudit dispositif ;
(c) l'envoi de l'image électronique à un moniteur distant via un moyen d'envoi (10) et un réseau numérique ;
(d) l'examen de la plante du pied par le moniteur distant, ledit examen de la plante du pied comprenant :
d1) la détection de petites plaies qui peuvent être présentes sur la plante du pied ;
d2) la comparaison de la plante du pied avec un examen précédent qui peut avoir été réalisé.

2. Procédé selon la revendication 1, dans lequel ladite image électronique comprend une photocopie couleur de la plante du pied.

3. Procédé selon la revendication 1, dans lequel ledit moyen d'enregistrement d'image comprend une caméra numérique.

4. Procédé selon la revendication 1, dans lequel ledit moyen d'envoi comprend un modem.

5. Procédé selon la revendication 1, dans lequel ledit dispositif comprend un moyen d'alarme pour alerter le patient à intervalles réguliers sur la nécessité d'un examen.
